# EUROPEAN PATENT APPLICATION

(11) **EP 1 402 872 A1**
(43) Date of publication of application: **31.03.2004**
(21) Application number: 03019441.9
(22) Date of filing: 28.08.2003
(51) Int. Cl.: A61K 6/083, A61K 6/00

(54) **Light-curable dental adhesive composition**

(30) Priority: 17.09.2002 JP 2002269957
(71) Applicant: DENTSPLY-SANKIN K.K., Ohtawara-shi, Tochigi 324-0036 (JP)
(72) Inventor: Hattori, Nobuyuki, Ohtawara-shi, Tochigi 324-0036 (JP)
(74) Representative: Steinmeister, Helmut

(57) **Abstract**

A light-curable dental adhesive composition contains a fluoride-releasing monomer that slowly releases fluoride ions over a long period of time, a first radical polymerizable monomer curable by photopolymerization upon irradiation with light and having a phosphoric acid group for bonding between members, a second radical polymerizable monomer curable by photopolymerization upon irradiation with light to bond between members, a photosensitizer for initiating the photopolymerization, a photopolymerization accelerator for accelerating curing of the first and second radical polymerizable monomers by irradiation with light, a filler for improving physical properties, and the balance consisting essentially of water and a dispersant. The filler is hydrophobic silicon dioxide having a primary particle average diameter of 8 to 13 nm and a specific surface area of 170±20 m²/g.

## Description

### BACKGROUND OF THE INVENTION

### 1. Field of the Invention

The present invention relates to a light-curable dental adhesive composition capable of firmly bonding to the dentin. More particularly, the present invention relates to a light-curable dental adhesive composition of the type composed of two liquids that are mixed together when it is going to be used. The viscosity of the light-curable dental adhesive composition is unlikely to decrease over a long period of time after the two liquids have been compounded together.

### 2. Description of Related Art

There have been proposed dental resin compositions capable of slowly releasing fluoride ions over a long period of time [see Japanese Patent Application Unexamined Publication (KOKAI) No. Hei 7-101819]. Liquid dental adhesive compositions composed of these resin materials, however, increase in viscosity when they are allowed to stand for a long period of time. Consequently, it may become impossible or difficult to use the adhesive compositions.

The present applicant proposed a one-part dental resin composition similarly capable of slowly releasing fluoride ions over a long period of time. When it is applied to the wet tooth surface, the composition increases in the degree of acidity and can exhibit the decalcification efficiency with respect to the dentin. Accordingly, the dental resin composition makes it possible to obtain the adhesion between the tooth surface and the adherend in a single step without the need for the pretreatment as required in the past (see Japanese Patent Application No. 2002-076787).

A liquid dental adhesive composition capable of slowly releasing fluoride ions over a long period of time, in particular, increases in viscosity when it is allowed to stand for a long time. Accordingly, the liquid dental adhesive composition may become impossible to use. Consequently, a dental clinic or the like that uses small amounts of liquid dental adhesive composition may have to throw away the composition left unspent. This results in an increased cost and is waste of resources.

### SUMMARY OF THE INVENTION

The present invention was made on the basis of the above-described technical background. Accordingly, an object of the present invention is to provide a light-curable dental adhesive composition usable for a long period of time with a minimal increase in viscosity.

To attain the above-described object, the present invention provides a light-curable dental adhesive composition containing a fluoride-releasing monomer that slowly releases fluoride ions over a long period of time, a first radical polymerizable monomer curable by photopolymerization upon irradiation with light and having a phosphoric acid group for bonding between members, a second radical polymerizable monomer curable by photopolymerization upon irradiation with light to bond between members, a photosensitizer for initiating the photopolymerization, a photopolymerization accelerator for accelerating curing of the first radical polymerizable monomer and the second radical polymerizable monomer by the irradiation with light, a filler for improving physical properties, and the balance consisting essentially of water and a dispersant. The filler is hydrophobic silicon dioxide having a primary particle average diameter of 8 to 13 nm and a specific surface area of 170±20 m²/g.

The light-curable dental adhesive composition according to the present invention can be given antisagging properties (thixotropic properties) while maintaining high flowability simply by mixing it with specific silicon dioxide having its surface made hydrophobic. Further, because it contains a fluoride-releasing monomer capable of slowly releasing fluoride ions over a long period of time, the light-curable dental adhesive composition can also exhibit the dental caries prevention effect by slowly releasing fluoride over a long period of time.

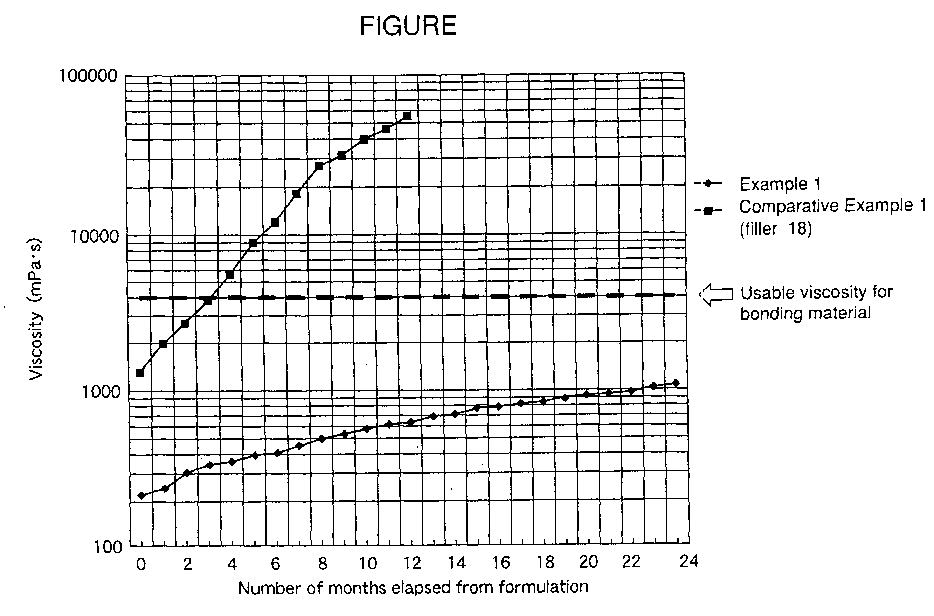

### BRIEF DESCRIPTION OF THE DRAWING

The attached sole figure is a graph showing the change of viscosity of Example 1 and Comparative Example 1 with passage of time in terms of the number of months.

### DETAILED DESCRIPTION OF THE INVENTION

### [Way of Using the Present Invention]

A typical way of using the light-curable dental adhesive composition according to the present invention is as follows. The light-curable dental adhesive composition is composed of two liquids that are mixed together when it is going to be used. One of the two liquids is a base component consisting essentially of the fluoride-releasing monomer, the first radical polymerizable monomer, the photosensitizer, and the photopolymerization accelerator. The other of the two liquids is an auxiliary component consisting essentially of the water, the dispersant, the second radical polymerizable monomer, and the filler. According to the knowledge of the present inventor, the light-curable dental adhesive composition can be given antisagging properties (thixotropic properties) while maintaining high flowability particularly by allowing the auxiliary component to contain the filler. However, the light-curable dental adhesive composition according to the present invention is not limited to the two-part type but may be of the one-part type. However, it is preferable to apply the present invention to the two-part type from the viewpoint of attaining even more effective results.

### [Fluoride-Releasing Monomer]

The fluoride-releasing monomer used in the present invention is preferably a cyclic phosphazene compound. The cyclic phosphazene compound is publicly known from the above-mentioned Japanese Patent Application Unexamined Publication (KOKAI) No. Hei 7-101819 and so forth. In general, it is expressed by the following chemical formula.

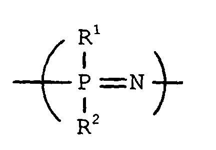

Any cyclic phosphazene compound having a constitutional unit expressed by the above-described chemical formula can be used in the present invention. For example, six-membered ring compounds and eight-membered ring compounds are usable in the present invention. It should be noted that at least one of R¹ and R² in the formula represents F in the cyclic phosphazene compound formed, and the remainder is the same or different group having a polymerizable double bond. Accordingly, R¹ and R² should not be limitatively construed as having an absolute meaning. The above-described group having a polymerizable double bond is preferably a (meth)acryloyloxyalkyl group, e.g. 2-(meth)acryloyloxymethoxy group, 2-(meth)acryloyloxyethoxy group, 2-(meth)acryloyloxypropoxy group, or 2-(meth)acryloyloxybutoxy group, and more preferably 2-(meth)acryloyloxyethoxy group.

Preferable examples of such cyclic phosphazene compounds are six-membered ring compounds of P₃N₃(F)ₙ[O(CH₂)₂COO(CH₃)C=CH₂]₆₋ₙ (n is any integer of 1 to 5), and eight-membered ring compounds of P₄N₄(F)ₘ[O(CH₂)₂COO(CH₃)C=CH₂]₈₋ₘ (m is any integer of 1 to 7).

These compounds can be obtained by allowing a six-membered ring compound of P₃N₃F₆ or an eight-membered ring compound of P₄N₄F₈, for example, to react with hydroxyethyl methacrylate. The method for this reaction is not particularly limited. An already-known method can be used for the reaction. For example, an organic base, e.g. pyridine or triethylamine, used as a dehydrofluorinating agent is allowed to react with the above-described reaction substance in an organic solvent, e.g. benzene or toluene, at about 50°C for 5 to 60 hours. The fluoride-releasing monomer in the present invention uses one or more of these compounds alone or in the form of a mixture.

When the total weight of the light-curable dental adhesive composition is 100% by weight, the amount of the fluoride-releasing monomer used in the present invention is 0.1 to 50.0% by weight, preferably 1.0 to 10.0% by weight.

### [First Radical Polymerizable Monomer Having Phosphoric Acid Group]

The first radical polymerizable monomer having a phosphoric acid group, which is used in the present invention, is a component for exhibiting the decalcification efficiency with respect to the dentin. When the light-curable dental adhesive composition according to the present invention, which contains the first radical polymerizable monomer having a phosphoric acid group, is applied to the tooth surface, this component dissociates from the composition, causing the degree of acidity to increase, and thus exhibiting the decalcification efficiency with respect to the dentin.

Examples of the first radical polymerizable monomer are phosphoric esters of (meth)acrylic acid and pyrophosphoric esters of (meth)acrylic acid. Examples of phosphoric esters of (meth)acrylic acid include (meth)acryloxymethyl phosphate, (meth) acryloxyethyl phosphate, (meth)acryloxypropyl phosphate, (meth)acryloxybutyl phosphate, (meth)acryloxypentyl phosphate, and (meth)acryloxyhexyl phosphate.

Examples of pyrophosphoric esters of (meth)acrylic acid include tetra(meth)acryloxyethyl pyrophosphate and di(meth)acryloxyethyl pyrophosphate. Among them, tetramethacryloxyethyl pyrophosphate is preferable. The first radical polymerizable monomer having a phosphoric acid group according to the present invention uses one or more of these substances alone or in the form of a mixture.

When the total weight of the light-curable dental adhesive composition is 100% by weight, the amount of the first radical polymerizable monomer having a phosphoric acid group used in the present invention is 5.0 to 80.0% by weight, preferably 30.0 to 80.0% by weight.

### [Second Radical Polymerizable Monomer]

The second radical polymerizable monomer used in the present invention is not indispensable for an adhesive, but it is a monomer compounded in addition to the above-described components to improve the function of the composition as an adhesive. Such a radical polymerizable monomer may be either a monofunctional monomer or a polyfunctional monomer.

Examples of the second radical polymerizable monomer include methyl (meth)acrylate, hydroxyalkyl (meth)acrylates, e.g. hydroxymethyl (meth)acrylate, hydroxyethyl (meth)acrylate, hydroxypropyl (meth)acrylate, and hydroxybutyl (meth)acrylate, ethylene glycol di(meth)acrylate, di- or tri- or tetra-ethylene glycol di(meth)acrylate, di(methacryloxyethyl)trimethyl hexamethylene diurethane [generally abbreviated as "UDMA"], dimethacrylates having a phenyl group, e.g. 2,2-bis(4-methacryloylethoxyphenyl) propane (Bis-MEPP), carboxylic acid vinyl esters, e.g. vinyl acetate, vinyl butyrate, and vinyl stearate, and ethylene unsaturated dicarboxylic acids, e.g. fumaric acid, maleic acid, and itaconic acid. Among these monomers, hydroxyl methacrylate is preferable. The second radical polymerizable monomer in the present invention uses one or more of these substances alone or in the form of a mixture.

When the total weight of the light-curable dental adhesive composition is 100% by weight, the amount of the second radical polymerizable monomer used in the present invention is 5.0 to 50.0% by weight, preferably 5.0 to 40.0% by weight.

### [Photosensitizer]

The photosensitizer used in the present invention is for initiating photopolymerization. The photosensitizer releases free radicals upon irradiation with light to exhibit a photosensitizing effect. It is preferable to use α-diketone. The kind of α-diketone is not particularly limited. Preferable examples include those which have a photosensitizing effect with respect to visible light, such as camphorquinone, benzil, biacetyl, 9,10-phenanthrenequinone, and naphthoquinone. Among them, camphorquinone is preferable.

When the total weight of the light-curable dental adhesive composition is 100% by weight, the amount of the photosensitizer used in the present invention is 0.1 to 10.0% by weight, preferably 0.1 to 1.0% by weight.

### [Photopolymerization Accelerator]

The photopolymerization accelerator used in the present invention is for accelerating curing of the first and second radical polymerizable monomers by irradiation with light. When the total weight of the light-curable dental adhesive composition is 100% by weight, the amount of the photopolymerization accelerator used in the present invention is 0.1 to 10.0% by weight, preferably 0.1 to 1.0% by weight.

The photopolymerization accelerator is preferably at least one selected from tertiary amine reducing agents. Specific examples include N,N-dimethylamino-P-toluidine, butyl ethanolamine, N,N-dimethylaminoethyl methacrylate, morpholinoethyl methacrylate, ethyl-P-(N,N-dimethylamino)benzoic acid, 2-methacryloxyethyl-P-(N,N-dimethylamino)benzoic acid, dimethylaminobenzoic acid, and esters of these components. One or more of these photopolymerization accelerators are used alone or in the form of a mixture.

### [Water-Soluble Organic Solvent and Water]

The water-soluble organic solvent and water used in the present invention are for uniformly dissolving or dispersing the fluoride-releasing monomer, the first radical polymerizable monomer having a phosphoric acid group, the second radical polymerizable monomer, the photopolymerization accelerator, the photosensitizer and the filler to facilitate the application of the composition to the tooth surface. In addition, the water-soluble organic solvent and water facilitate the contact between the tooth surface and the light-curable dental adhesive composition according to the present invention and also facilitate mixing and hence allow the ionization of the phosphoric acid group to proceed, thereby exhibiting the decalcification function. Furthermore, the water-soluble organic solvent and water serve to ensure a moderate thickness for an adhesive layer formed on the dentin surface after the polymerization reaction has proceeded thereon.

Examples of water-soluble organic solvents usable are alcohols such as ethanol, 1-propanol, isopropyl alcohol, diethylene glycol, and triethylene glycol, and ketones such as acetone and methyl ethyl ketone. Among them, ethanol is preferable from the viewpoint of safety because the composition containing the water-soluble organic solvent is used in the living body. Regarding water, purified water is preferable from the viewpoint of preventing impurities from mixing into the composition.

There are no particular restrictions on the water-soluble organic solvent and water used in the present invention. It is possible to use any water-soluble organic solvent and water that can dissolve or disperse the components of the light-curable dental adhesive composition according to the present invention. It is preferable that the amount of the water-soluble organic solvent and water should be roughly in the range of from 30 to 90 parts by weight with respect to all the components of the light-curable dental adhesive composition according to the present invention.

### [Filler]

The filler used in the present invention is preferably hydrophobic silicon dioxide having a primary particle average diameter of 8 to 13 nm and a specific surface area of 170±20 m²/g. The hydrophobic silicon dioxide used in the present invention has various shapes, such as the shapes of sphere, elliptical sphere, hollow sphere and hollow elliptical sphere. Accordingly, the size of the specific surface area is not mathematically proportional to the average diameter of the primary particles. However, the above-described primary particle average diameter and specific surface area are substantially preferable. The surface group of the silicon dioxide is preferably (CH₃)₂.

By being mixed into the light-curable dental adhesive composition according to the present invention, the filler in the present invention allows antisagging properties (thixotropic properties) to be kept for a long period of time while maintaining high flowability. When the total weight of the light-curable dental adhesive composition is 100% by weight, the amount of the filler used in the present invention is 0.5 to 15.0% by weight, preferably 1.0 to 10.0% by weight.

It should be noted that the above-described cyclic phosphazene compound used in the present invention may be employed as an organic filler in such a way that the cyclic phosphazene compound is previously polymerized to obtain a cured material and this is powdered. Further, another inorganic or organic filler may be added in addition to the above-described filler to improve wear resistance, for example, provided that high flowability can be maintained and antisagging properties (thixotropic properties) can be kept for a long period of time.

### [Other Components]

Other components may be added to the light-curable dental adhesive composition according to the present invention within the range in which the adhesive properties are not impaired. For example, polyacrylic acid or the like may be added as a polymer component to improve bond strength. It is also possible to add a polymerization inhibitor, a coloring agent, an ultraviolet light absorber, etc. according to need. Compositions additionally containing these components are also included in the scope of the present invention. The light-curable dental adhesive composition according to the present invention is usable as a dental adhesive, e.g. a resin for partial dentures, a denture rebasing or relining material, a rigid resin for crowns, a filling resin, a sealant for dental caries prevention, an orthodontic bracket adhesive, or a banding cement.

### EXAMPLES

The present invention will be described below by way of examples. It should be noted, however, that the present invention is not limited to these examples. Tables 1 and 2 below show the composition of each example of a fluoride-releasing dental adhesive according to the present invention. The fluoride-releasing dental adhesive is composed of two liquids that are mixed together when it is going to be used.

Silica, i.e. silicon dioxide, is hydrophobic silicon dioxide. "AEROSIL R974" (trade name; available from Nippon Aerosil Co., Ltd.; the seat of the head office: Shinjuku-ku, Tokyo, Japan) was used as hydrophobic silicon dioxide. The main specifications are as follows. The primary particle average diameter is about 12 (nm). The specific surface area is 170±20 (m²/g). The pH value in a 4% water solution is from 4.0 to 5.5. The carbon content is about 1%. The apparent specific gravity is about 50 (g/l). The surface group of the silicon dioxide is (CH₃)₂. The hydrophobic silicon dioxide used in this example had been made hydrophobic by a chemical reaction taking place on the solid surface. The methanol wettability, which represents hydrophobic nature, is ">35".

In the following Examples 1 to 7, the changes in viscosity of examples shown below were examined for one month under the environment of 40°C as an accelerated test for shortening the experiment period. In the accelerated test, the period of one month was regarded as 24 months. The upper limit of the viscosity at which each sample was usable as a dental adhesive was determined to be 4000 mPa·s. Samples whose viscosity remained within the upper limit value for one month were determined to be examples.

**Table 1**

| Components | Ex.1 Aux. | Base | Ex.2 Aux. | Base | Ex.3 Aux. | Base | Ex.4 Aux. | Base |
|---|---|---|---|---|---|---|---|---|
| Pyrophosphoric ester | 0.00 | 65.00 | 0.00 | 70.00 | 0.00 | 70.00 | 0.00 | 68.00 |
| Cyclic phosphazene monomer | 0.00 | 5.00 | 0.00 | 5.00 | 0.00 | 3.00 | 0.00 | 3.00 |
| Ethanol | 30.00 | 0.00 | 26.00 | 0.00 | 26.00 | 0.00 | 32.50 | 0.00 |
| Purified water | 40.00 | 0.00 | 30.00 | 0.00 | 25.00 | 0.00 | 25.00 | 0.00 |
| N,N-dimethyl amine | 0.00 | 1.00 | 0.00 | 0.00 | 0.00 | 3.40 | 0.00 | 3.40 |
| N,N-dimethyl amino-P-toluidine | | | 0.00 | 5.00 | 0.00 | 1.00 | 1.50 | 1.00 |
| Camphorquinone | 0.00 | 0.50 | 0.00 | 0.50 | 0.00 | 0.60 | 0.00 | 0.60 |
| Benzil | | | | | | | | |
| Urethane dimethacrylate | 0.00 | 28.50 | 0.00 | 10.00 | 0.00 | 12.00 | 0.00 | 12.00 |
| Triethylene glycol dimethacrylate | | | 0.00 | 9.50 | 0.00 | 10.00 | 0.00 | 12.00 |
| 2-hydroxy methacrylate | 25.00 | 0.00 | 40.00 | 0.00 | 46.00 | 0.00 | 36.00 | 0.00 |
| Filler | 5.00 | 0.00 | 4.00 | 0.00 | 3.00 | 0.00 | 5.00 | 0.00 |
| TOTAL | 100.00 | 100.00 | 100.00 | 100.00 | 100.00 | 100.00 | 100.00 | 100.00 |

| Bond Strength | | | | | | | | |
|---|---|---|---|---|---|---|---|---|
| Enamel | 17.00 | | 12.00 | | 10.50 | | 14.00 | |
| Dentin | 20.00 | | 15.00 | | 14.00 | | 13.00 | |

**Table 2**

| | Ex.5 Aux. | Base | Ex.6 Aux. | Base | Ex.7 Aux. | Base |
|---|---|---|---|---|---|---|
| Pyrophosphoric ester | 0.00 | 65.00 | 0.00 | 70.00 | 0.00 | 67.50 |
| Cyclic phosphazene monomer | 0.00 | 5.00 | 0.00 | 5.00 | 0.00 | 3.00 |
| Ethanol | 30.00 | 0.00 | 56.00 | 0.00 | 46.00 | 0.00 |
| Purified water | 40.00 | 0.00 | 0.00 | 0.00 | 5.00 | 0.00 |
| N,N-dimethyl amine | 0.00 | 1.00 | 0.00 | 0.00 | 0.00 | 3.40 |
| N,N-dimethylamino-P-toluidine | | | 0.00 | 5.00 | 0.00 | 1.00 |
| Camphorquinone | 0.00 | 0.20 | 0.00 | 0.50 | 0.00 | 0.10 |
| Benzil | 0.00 | 0.30 | | | | |
| Urethane dimethacrylate | 0.00 | 28.50 | 0.00 | 10.00 | 0.00 | 5.00 |
| Triethylene glycol dimethacrylate | | | 0.00 | 9.50 | 0.00 | 20.00 |
| 2-hydroxy methacrylate | 25.00 | 0.00 | 40.00 | 0.00 | 46.00 | 0.00 |
| Filler | 5.00 | 0.00 | 4.00 | 0.00 | 3.00 | 0.00 |
| TOTAL | 100.00 | 100.00 | 100.00 | 100.00 | 100.00 | 100.00 |

| Bond Strength | | | | | | |
|---|---|---|---|---|---|---|
| Enamel | 13.00 | | 10.00 | | 10.00 | |
| Dentin | 14.00 | | 12.00 | | 12.00 | |

### COMPARATIVE EXAMPLES

Table 3 below shows Comparative Examples 1 and 2. In these examples, the upper limit of the viscosity at which each sample was usable as a dental adhesive was determined to be 4000 mPa·s. Samples whose viscosity exceeded the upper limit value in one month as a result of the accelerated test were judged to be inapplicable and determined to be comparative examples. As the inorganic filler, silicon dioxide "AEROSIL" (trade name), available from Nippon Aerosil Co., Ltd. (the seat of the head office: Shinjuku-ku, Tokyo, Japan) was used.

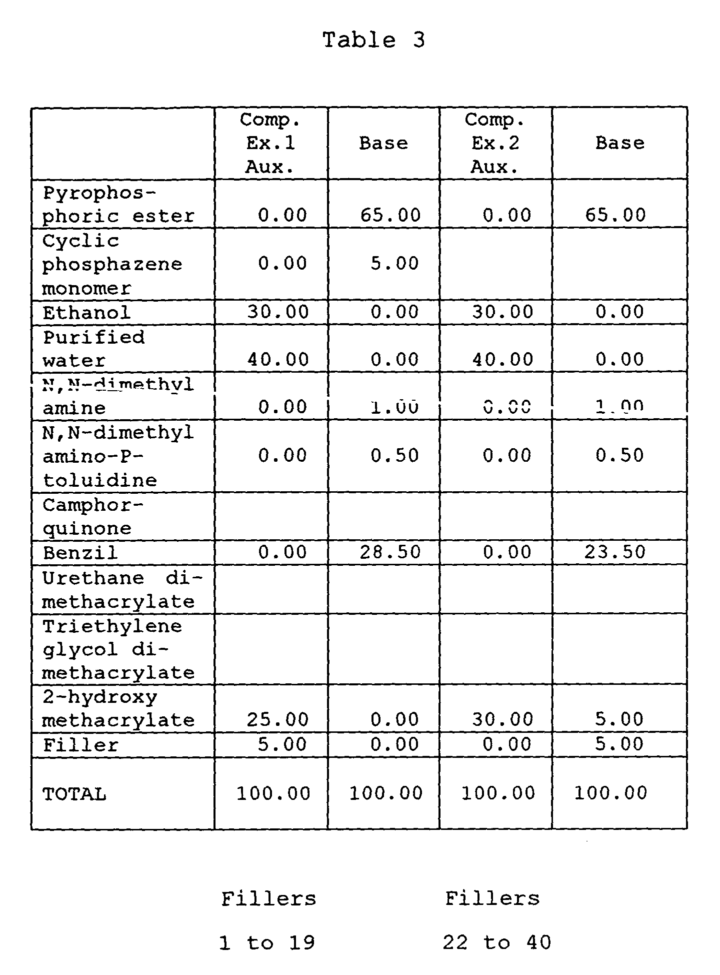

The specifications of the inorganic fillers 1 to 19 used in Comparative Example 1 are as shown in Tables 4 to 7 below.

**Table 4**

| Specifications of fillers 1 to 6 | | | | | | |
|---|---|---|---|---|---|---|
| Items | Spec. of filler 1 | Spec. of filler 2 | Spec. of filler 3 | Spec. of filler 4 | Spec. of filler 5 | Spec. of filler 6 |
| Surface treatment | Not done | Not done | Not done | Not done | Not done | Not done |
| Hydrophilic/hydrophobic | Hydrophilic | Hydrophilic | Hydrophilic | Hydrophilic | Hydrophilic | Hydrophilic |
| Specific surface area | 50.0 | 90.0 | 130.0 | 200.0 | 200.0 | 200.0 |
| Particle diameter | 30.0 | 20.0 | 16.0 | 12.0 | 12.0 | 12.0 |
| Apparent specific gravity | 50.0 | 50.0 | 50.0 | 50.0 | 100.0 | 30.0 |

**Table 5**

| Specifications of fillers 7 to 12 | | | | | | |
|---|---|---|---|---|---|---|
| Items | Spec. of filler 7 | Spec. of filler 8 | Spec. of filler 9 | Spec. of filler 10 | Spec. of filler 11 | Spec. of filler 12 |
| Surface treatment | Not done | Not done | Not done | Not done | Not done | Not done |
| Hydrophilic/hydrophobic | Hydrophilic | Hydrophilic | Hydrophilic | Hydrophilic | Hydrophilic | Hydrophilic |
| Specific surface area | 300.0 | 300.0 | 380.0 | 50.0 | 200.0 | 80.0 |
| Particle diameter | 7.0 | 7.0 | 7.0 | 40.0 | 40.0 | 30.0 |
| Apparent specific gravity | 50.0 | 30.0 | 50.0 | 130.0 | 60.0 | 60.0 |

**Table 6**

| Specifications of fillers 13 to 17 | | | | | |
|---|---|---|---|---|---|
| Items | Spec. of filler 13 | Spec. of filler 14 | Spec. of filler 15 | Spec. of filler 16 | Spec. of filler 17 |
| Surface treatment | Not done | Dimethylsilyl | Dimethylsilyl | Dimethylsilyl | Dimethylsilicone oil |
| Hydrophilic/hydrophobic | Hydrophilic | Hydrophobic | Hydrophobic | Hydrophobic | Hydrophobic |
| Specific surface area | 170.0 | 110.0 | 110.0 | 110.0 | 100.0 |
| Particle diameter | 30.0 | 16.0 | 16.0 | 16.0 | 14.0 |
| Apparent specific gravity | 50.0 | 50.0 | 90.0 | 30.0 | 50.0 |

**Table 7**

| Specifications of fillers 18 to 19 | | |
|---|---|---|
| Items | Spec. of filler 18 | Spec. of filler 19 |
| Surface treatment | Trimethylsilyl | Trimethylsilyl |
| Hydrophilic/hydrophobic | Hydrophobic | Hydrophobic |
| Specific surface area | 260.0 | 220.0 |
| Particle diameter | 7.0 | 7.0 |
| Apparent specific gravity | 50.0 | 50.0 |

The specifications of the inorganic fillers 22 to 40 used in Comparative Example 2 are as shown in Tables 8 to 11 below.

**Table 8**

| Specifications of fillers 22 to 27 | | | | | | |
|---|---|---|---|---|---|---|
| Items | Spec. of filler 22 | Spec. of filler 23 | Spec. of filler 24 | Spec. of filler 25 | Spec. of filler 26 | Spec. of filler 27 |
| Surface treatment | Not done | Not done | Not done | Not done | Not done | Not done |
| Hydrophilic/hydrophobic | Hydrophilic | Hydrophilic | Hydrophilic | Hydrophilic | Hydrophilic | Hydrophilic |
| Specific surface area | 50.0 | 90.0 | 130.0 | 200.0 | 200.0 | 200.0 |
| Particle diameter | 30.0 | 20.0 | 16.0 | 12.0 | 12.0 | 12.0 |
| Apparent specific gravity | 50.0 | 50.0 | 50.0 | 50.0 | 100.0 | 30.0 |

**Table 9**

| Specifications of fillers 28 to 33 | | | | | | |
|---|---|---|---|---|---|---|
| Items | Spec. of filler 28 | Spec. of filler 29 | Spec. of filler 30 | Spec. of filler 31 | Spec. of filler 32 | Spec. of filler 33 |
| Surface treatment | Not done | Not done | Not done | Not done | Not done | Not done |
| Hydrophilic/hydrophobic | Hydrophilic | Hydrophilic | Hydrophilic | Hydrophilic | Hydrophilic | Hydrophilic |
| Specific surface area | 300.0 | 300.0 | 380.0 | 50.0 | 200.0 | 80.0 |
| Particle diameter | 7.0 | 7.0 | 7.0 | 40.0 | 40.0 | 30.0 |
| Apparent specific gravity | 50.0 | 30.0 | 50.0 | 130.0 | 60.0 | 60.0 |

**Table 10**

| Specifications of fillers 34 to 38 | | | | | |
|---|---|---|---|---|---|
| Items | Spec. of filler 34 | Spec. of filler 35 | Spec. of filler 36 | Spec. of filler 37 | Spec. of filler 38 |
| Surface treatment | Not done | Dimethylsilyl | Dimethylsilyl | Dimethylsilyl | Dimethylsilicone oil |
| Hydrophilic/hydrophobic | Hydrophilic | Hydrophobic | Hydrophobic | Hydrophobic | Hydrophobic |
| Specific surface area | 170.0 | 110.0 | 110.0 | 110.0 | 100.0 |
| Particle diameter | 30.0 | 16.0 | 16.0 | 16.0 | 14.0 |
| Apparent specific gravity | 50.0 | 50.0 | 90.0 | 30.0 | 50.0 |

**Table 11**

| Specifications of fillers 39 to 40 | | |
|---|---|---|
| Items | Spec. of filler 39 | Spec. of filler 40 |
| Surface treatment | Trimethylsilyl | Trimethylsilyl |
| Hydrophilic/ hydrophobic | Hydrophobic | Hydrophobic |
| Specific surface area | 260.0 | 220.0 |
| Particle diameter | 7.0 | 7.0 |
| Apparent specific gravity | 50.0 | 50.0 |

It has become clear from the above-described Comparative Examples 1 and 2 that the shelf stability is not good when a fluoride-releasing monomer is used in coexistence with an inorganic filler other than those used in Examples 1 to 7.

The attached Figure is a graph showing the change of viscosity with passage of time in terms of the number of months. The graph shows data concerning the above-described Example 1 and Comparative Example 1, by way of example. The fluoride-releasing dental adhesive using silicon dioxide having its surface made hydrophobic, i.e. "AEROSIL R974", still maintains a usable viscosity even after 24 months has elapsed. The test was carried out in the same way as in the accelerated test for the examples.

### [Advantageous Effect of the Invention]

As has been detailed above, the light-curable dental adhesive composition according to the present invention can be given antisagging properties (thixotropic properties) while maintaining high flowability simply by mixing it with specific silicon dioxide having its surface made hydrophobic.

## Claims

1. A light-curable dental adhesive composition comprising:
a fluoride-releasing monomer that slowly releases fluoride ions over a long period of time;
a first radical polymerizable monomer curable by photopolymerization upon irradiation with light and having a phosphoric acid group for bonding between members;
a second radical polymerizable monomer curable by photopolymerization upon irradiation with light to bond between members;
a photosensitizer for initiating said photopolymerization;
a photopolymerization accelerator for accelerating curing of said first radical polymerizable monomer and second radical polymerizable monomer by said irradiation with light;
a filler for improving physical properties; and
the balance consisting essentially of water and a dispersant;
wherein said filler is hydrophobic silicon dioxide having a primary particle average diameter of from 8 nm to 13 nm and a specific surface area of 170±20 m²/g.

2. A light-curable dental adhesive composition according to claim 1, which is composed of two liquids that are mixed together when said light-curable dental adhesive composition is going to be used, one of said two liquids being a base component consisting essentially of said fluoride-releasing monomer, said first radical polymerizable monomer, said photosensitizer, and said photopolymerization accelerator, the other of said two liquids being an auxiliary component consisting essentially of said water, said dispersant, said second radical polymerizable monomer, and said filler.

3. A light-curable dental adhesive composition according to claim 1 or 2, wherein said fluoride-releasing monomer is a cyclic phosphazene compound;
said first radical polymerizable monomer being at least one of a phosphoric ester of (meth)acrylic acid and a pyrophosphoric ester of (meth)acrylic acid;
said second radical polymerizable monomer being at least one selected from the group consisting of methyl (meth)acrylate, hydroxyalkyl (meth)acrylates, ethylene glycol di(meth)acrylate, di- or tri- or tetra-ethylene glycol di(meth)acrylate, di(methacryloxyethyl)trimethyl hexamethylene diurethane, dimethacrylates having a phenyl group, carboxylic acid vinyl esters, and ethylene unsaturated dicarboxylic acids;
said photosensitizer being at least one selected from the group consisting of camphorquinone, benzil, biacetyl, 9,10-phenanthrenequinone, and naphthoquinone; and
said photopolymerization accelerator being at least one selected from tertiary amine reducing agents.

4. A light-curable dental adhesive composition according to claim 1 or 2, wherein a surface group of said silicon dioxide is (CH₃)₂.

5. A light-curable dental adhesive composition according to claim 1 or 2, which contains:
from 0.1 to 50.0% by weight of said fluoride-releasing monomer;
from 5.0 to 80.0% by weight of said first radical polymerizable monomer;
from 5.0 to 50.0% by weight of said second radical polymerizable monomer;
from 0.1 to 10.0% by weight of said photosensitizer;
from 0.1 to 10.0% by weight of said photopolymerization accelerator;
from 0.5 to 15.0% by weight of said filler; and
the balance consisting essentially of said water and said dispersant.

6. A light-curable dental adhesive composition according to claim 1 or 2, which contains:
from 1.0 to 10.0% by weight of said fluoride-releasing monomer;
from 30.0 to 80.0% by weight of said first radical polymerizable monomer;
from 5.0 to 40.0% by weight of said second radical polymerizable monomer;
from 0.1 to 1.0% by weight of said photosensitizer;
from 0.1 to 1.0% by weight of said photopolymerization accelerator;
from 1.0 to 10.0% by weight of said filler; and
the balance consisting essentially of said water and said dispersant.
